# EUROPÄISCHE PATENTANMELDUNG

(11) **EP 2 923 760 A1**
(43) Veröffentlichungstag der Anmeldung: **30.09.2015**
(21) Anmeldenummer: 15157721.0
(22) Anmeldetag: 05.03.2015
(51) Int. Cl.: B01L 3/00, C12Q 1/68, G01N 33/543

(54) **CHIPLABOR-KARTUSCHE FÜR EIN MIKROFLUIDISCHES SYSTEM ZUM ANALYSIEREN EINER PROBE BIOLOGISCHEN MATERIALS, MIKROFLUIDISCHES SYSTEM ZUM ANALYSIEREN EINER PROBE BIOLOGISCHEN MATERIALS SOWIE VERFAHREN UND VORRICHTUNG ZUM ANALYSIEREN EINER PROBE BIOLOGISCHEN MATERIALS**

(30) Priorität: 27.03.2014 DE 102014205728
(71) Anmelder: ROBERT BOSCH GMBH, 70442 Stuttgart (DE)
(72) Erfinder: Behrens, Holger, 70192 Stuttgart (DE)

(57) **Zusammenfassung**

Die Erfindung betrifft eine Chiplabor-Kartusche (310) für ein mikrofluidisches System (300) zum Analysieren einer Probe biologischen Materials. Dabei weist die Chiplabor-Kartusche (310) eine Probenaufnahmekammer (320) auf, die eine Einbringöffnung (322) zum Einbringen einer Probe biologischen Materials in die Chiplabor-Kartusche (310), einen ersten Probenauslass (324) zum Auslassen einer ersten Teilmenge der Probe aus der Probenaufnahmekammer (320) und einen zweiten Probenauslass (326) zum Auslassen einer zweiten Teilmenge der Probe aus der Probenaufnahmekammer (320) aufweist. Auch weist die Chiplabor-Kartusche (310) eine erste Probenbearbeitungseinrichtung (330) auf, die fluidisch mit dem ersten Probenauslass (324) der Probenaufnahmekammer (320) verbunden ist. Hierbei ist die erste Probenbearbeitungseinrichtung (330) ausgebildet, um einen ersten Vorbereitungsprozess und einen ersten Analyseprozess an der ersten Teilmenge der Probe durchzuführen. Ferner weist die Chiplabor-Kartusche (310) eine zweite Probenbearbeitungseinrichtung (340) auf, die fluidisch mit dem zweiten Probenauslass (326) der Probenaufnahmekammer (320) verbunden und fluidisch von der ersten Probenbearbeitungseinrichtung (330) getrennt ist. Dabei ist die zweite Probenbearbeitungseinrichtung (340) ausgebildet, um einen zweiten Vorbereitungsprozess und einen zweiten Analyseprozess an der zweiten Teilmenge der Probe durchzuführen.

## Beschreibung

### Stand der Technik

Die vorliegende Erfindung bezieht sich auf eine Chiplabor-Kartusche für ein mikrofluidisches System zum Analysieren einer Probe biologischen Materials, auf ein mikrofluidisches System zum Analysieren einer Probe biologischen Materials, auf ein Verfahren zum Analysieren einer Probe biologischen Materials, auf eine entsprechende Vorrichtung sowie auf ein entsprechendes Computerprogramm.

Chiplabor-Produkte bzw. Lab-on-Chip-Produkte (LOC-Produkte) werden insbesondere eingesetzt, um bestehende und bekannte, manuelle Assays zu automatisieren. Dabei kann ein Ablauf eines Assays, z. B. zur Diagnostik von antibiotikaresistenten Bakterien, auf eine Lab-on-Chip-Plattform übertragen werden. Die US2001/0036672 offenbart eine integrierte Nukleinsäure-Diagnostikvorrichtung.

### Offenbarung der Erfindung

Vor diesem Hintergrund werden mit dem hier vorgestellten Ansatz eine Chiplabor-Kartusche für ein mikrofluidisches System zum Analysieren einer Probe biologischen Materials, ein mikrofluidisches System zum Analysieren einer Probe biologischen Materials, ein Verfahren zum Analysieren einer Probe biologischen Materials, weiterhin eine Vorrichtung, die dieses Verfahren verwendet, sowie schließlich ein entsprechendes Computerprogramm gemäß den Hauptansprüchen vorgestellt. Vorteilhafte Ausgestaltungen ergeben sich aus den jeweiligen Unteransprüchen und der nachfolgenden Beschreibung.

Gemäß Ausführungsformen der Erfindung können insbesondere parallele Assayabläufe mit einer Probe innerhalb einer Kartusche in einem Lab-on-Chip-System ermöglicht werden. So können beispielsweise in einer Lab-on-Chip-Kartusche basierend auf einer Probe mehrere Assays parallel komplett prozessiert werden. Für eine solche parallele Prozesssierung kann die Probe insbesondere auf mehrere, parallele, fluidische Pfade innerhalb der Kartusche aufgeteilt werden. Hierbei kann eine Probe beispielsweise nach dem Einbringen des Swobs and anschließenden Abwaschen der Erreger vom Swob aufgeteilt und anschließend auf der Kartusche in mehreren fluidischen Zweigen bzw. Pfaden parallel prozessiert werden. Diese Prozesssierung ist beispielsweise bei Lab-on-Chip-Kartuschen mit einem planaren Fluidlayer möglich. In einem solchen Fluidnetzwerk können Kammern und Kanäle nebeneinander angeordnet sein. Des Weiteren können Ventile und Pumpen der Fluidpfade unabhängig voneinander angesteuert werden.

Vorteilhafterweise kann gemäß Ausführungsformen der Erfindung beispielsweise eine Probe mehrfach parallel prozessiert werden, wodurch ein Umfang therapeutisch relevanter Informationen gesteigert werden kann, wie beispielsweise Erreger- und Hormoninformation oder Aussage über tote und lebende Zellen etc. Eine Handhabung im Labor kann vereinfacht werden, da die Probe nur einmal in die Kartusche eingegeben und innerhalb der Kartusche aufgeteilt zu werden braucht. Ferner können dadurch Durchführungsfehler minimiert werden. Eine solche parallele Prozesssierung mehrerer kompletter Assays macht auch einen Einsatz von mehreren Kartuschen überflüssig, wodurch Kosten und Aufwand bei einer Bearbeitung im Labor eingespart werden können.

Es wird eine Chiplabor-Kartusche für ein mikrofluidisches System zum Analysieren einer Probe biologischen Materials vorgestellt, wobei die Chiplabor-Kartusche folgende Merkmale aufweist:
eine Probenaufnahmekammer, die eine Einbringöffnung zum Einbringen einer Probe biologischen Materials in die Chiplabor-Kartusche, einen ersten Probenauslass zum Auslassen einer ersten Teilmenge der Probe aus der Probenaufnahmekammer und zumindest einen zweiten Probenauslass zum Auslassen zumindest einer zweiten Teilmenge der Probe aus der Probenaufnahmekammer aufweist;
eine erste Probenbearbeitungseinrichtung, die fluidisch mit dem ersten Probenauslass der Probenaufnahmekammer verbunden ist, wobei die erste Probenbearbeitungseinrichtung ausgebildet ist, um einen ersten Vorbereitungsprozess und einen ersten Analyseprozess an der ersten Teilmenge der Probe durchzuführen; und
eine zweite Probenbearbeitungseinrichtung, die fluidisch mit dem zweiten Probenauslass der Probenaufnahmekammer verbunden und fluidisch von der ersten Probenbearbeitungseinrichtung getrennt ist, wobei die zweite Probenbearbeitungseinrichtung ausgebildet ist, um einen zweiten Vorbereitungsprozess und einen zweiten Analyseprozess an der zweiten Teilmenge der Probe durchzuführen.

Die Chiplabor-Kartusche kann als eine mikrofluidische Kartusche ausgeformt sein. Insbesondere kann die Chiplabor-Kartusche für Lab-on-Chip-Produkte und Medizinprodukte geeignet sein, welche Proben aufnehmen und anschließend automatisch weiterverarbeiten. Bei der Chiplabor-Kartusche kann es sich um einen Chip, insbesondere mit mehreren Schichten, aus einem Polymermaterial handeln. Dabei kann die Chiplabor-Kartusche als Teil eines mikrofluidischen Systems ausgeführt sein. Bei dem mikrofluidischen System kann es sich um ein analytisches System handeln, insbesondere ein mikrofluidisches Lab-on-Chip-System bzw. Chiplabor-System zur medizinischen Diagnostik, mikrobiologischen Diagnostik oder Umweltanalytik. Als Probe biologischen Materials kann eine zu analysierende Flüssigkeit, typischerweise eine flüssige oder verflüssigte Patientenprobe, z. B. Blut, Urin, Stuhl, Sputum, Liquor, Lavage, ein ausgespülter Abstrich oder eine verflüssigte Gewebeprobe, oder eine Probe eines nichtmenschlichen Materials bezeichnet werden. In der Probe können Zellen enthalten sein, wie beispielsweise menschliche Zellen, z. B. Blutzellen oder dergleichen, tierische Zellen oder pathogene Zellen bzw. Pathogene, z. B. Viren oder Mikroorganismen, wie z. B. Bakterien oder Pilze, umfassen, die DNA und/oder RNA, d. h. Nukleinsäuremoleküle aufweisen.

Gemäß einer Ausführungsform kann die erste Probenbearbeitungseinrichtung Bearbeitungsmittel aufweisen, die ausgebildet sind, um als ersten Vorbereitungsprozess zumindest einen Teilschritt des Aufbereitens, Aufschließens und zusätzlich oder alternativ Aufreinigens der ersten Teilmenge der Probe durchzuführen und zusätzlich oder alternativ als ersten Analyseprozess zumindest einen Teilschritt des Durchführens eines Immunassays, eines Molekularassays und zusätzlich oder alternativ einer Polymerasekettenreaktion an der ersten Teilmenge der Probe durchzuführen. Die erste Probenbearbeitungseinrichtung kann als Bearbeitungsmittel zumindest einen Fluidkanal, zumindest eine Kammer bzw. Kavität, zumindest eine Fördereinrichtung, zumindest ein Ventil und zusätzlich oder alternativ zumindest ein vorgelagertes Reagens aufweisen. Eine solche Ausführungsform bietet den Vorteil, dass eine parallele Bearbeitung einer Probe in zwei voneinander unabhängigen, fluidischen Pfaden auf einer Kartusche ermöglicht wird.

Auch kann die zweite Probenbearbeitungseinrichtung Bearbeitungsmittel aufweisen, die ausgebildet sind, um als zweiten Vorbereitungsprozess zumindest einen Teilschritt des Aufbereitens, Aufschließens und zusätzlich oder alternativ Aufreinigens der zweiten Teilmenge der Probe durchzuführen und zusätzlich oder alternativ als zweiten Analyseprozess zumindest einen Teilschritt des Durchführens eines Immunassays, eines Molekularassays und zusätzlich oder alternativ einer Polymerasekettenreaktion an der zweiten Teilmenge der Probe durchzuführen. Die zweite Probenbearbeitungseinrichtung kann als Bearbeitungsmittel zumindest einen Fluidkanal, zumindest eine Kammer bzw. Kavität, zumindest eine Fördereinrichtung, zumindest ein Ventil und zusätzlich oder alternativ zumindest ein vorgelagertes Reagens aufweisen. Eine solche Ausführungsform bietet den Vorteil, dass eine parallele Bearbeitung einer Probe in zwei voneinander unabhängigen, fluidischen Pfaden auf einer Kartusche ermöglicht wird.

Ferner kann sich der erste Vorbereitungsprozess von dem zweiten Vorbereitungsprozess in zumindest einem Teilschritt unterscheiden. Auch kann zusätzlich oder alternativ sich der erste Analyseprozess von dem zweiten Analyseprozess in zumindest einem Teilschritt unterscheiden. Hierbei können sich die Probenbearbeitungseinrichtungen in zumindest einem Bearbeitungsmittel unterscheiden. Die Prozesssierung innerhalb der Fluidpfade bzw. Probenbearbeitungseinrichtung kann sich insbesondere in der Art des Assays unterscheiden. Eine solche Ausführungsform bietet den Vorteil, dass eine parallele Probenbearbeitung mittels unterschiedlicher Assays auf einer Kartusche ermöglicht wird. Da sich eine Prozesssierung innerhalb solcher parallel geschalteter Fluidpfade sich in der Art des Assays unterscheiden kann, ist eine Aussagekraft einer Diagnose erweitert, die mit einer einzelnen Kartusche möglich ist. Dies ist vor allem deswegen vorteilhaft, da somit auch Parameter einer Probe untersucht werden können, die grundlegend unterschiedliche Nachweisverfahren benötigen, wie beispielsweise ein paralleler Nachweis von DNA und Proteinen.

Zudem können eine erste Ergebniskammer zum Beinhalten zumindest eines ersten Analyten, der durch den ersten Vorbereitungsprozess und den ersten Analyseprozess mittels der ersten Probenbearbeitungseinrichtung aus der ersten Teilmenge der Probe gewinnbar ist, und eine zweite Ergebniskammer zum Beinhalten zumindest eines zweiten Analyten, der durch den zweiten Vorbereitungsprozess und den zweiten Analyseprozess mittels der zweiten Probenbearbeitungseinrichtung aus der zweiten Teilmenge der Probe gewinnbar ist, vorgesehen sein. Somit kann sich die erste Probenbearbeitungseinrichtung als ein erster fluidischer Pfad von dem ersten Probenauslass zu der ersten Ergebniskammer erstrecken. Ferner kann sich die zweite Probenbearbeitungseinrichtung als ein zweiter fluidischer Pfad von dem zweiten Probenauslass zu der zweiten Ergebniskammer erstrecken. Die Ergebniskammern können ausgeformt und ausgebildet sein, um von innerhalb oder außerhalb der Chiplabor-Kartusche hinsichtlich zumindest einer Eigenschaft der Analyten, insbesondere optisch, auswertbar zu sein. Eine solche Ausführungsform bietet den Vorteil, dass eine Auswertung der Analyseergebnisse vereinfacht wird. Hierbei kann ein direkter Vergleich der Analyten aus den parallelen Bearbeitungspfaden der Probe erfolgen.

Auch wird ein mikrofluidisches System zum Analysieren einer Probe biologischen Materials vorgestellt, wobei das mikrofluidische System folgende Merkmale aufweist:
eine Ausführungsform der vorstehend genannten Chiplabor-Kartusche; und
eine Auswerteeinrichtung zum Auswerten der mittels der Chiplabor-Kartusche analysierten Probe, wobei die Auswerteeinrichtung ausgebildet ist, um zumindest einen ersten Analyten, der durch den ersten Vorbereitungsprozess und den ersten Analyseprozess mittels der ersten Probenbearbeitungseinrichtung aus der ersten Teilmenge der Probe gewinnbar ist, und zumindest einem zweiten Analyten, der durch den zweiten Vorbereitungsprozess und den zweiten Analyseprozess mittels der zweiten Probenbearbeitungseinrichtung aus der zweiten Teilmenge der Probe gewinnbar ist, auszuwerten.

In Verbindung mit dem mikrofluidischen System kann eine Ausführungsform der vorstehend genannten Chiplabor-Kartusche vorteilhaft eingesetzt bzw. verwendet werden, um eine Probe biologischen Materials zu analysieren bzw. zu analysieren . Das mikrofluidische System kann ausgebildet sein, um die Chiplabor-Kartusche aufzunehmen bzw. mit derselben zumindest fluidisch und mechanisch koppelbar zu sein. Die Auswerteeinrichtung kann ausgebildet sein, um die Analyten hinsichtlich zumindest einer Eigenschaft, insbesondere optisch, auszuwerten. Das mikrofluidische System kann ferner zumindest eine Ansteuereinrichtung zum Ansteuern der Probenbearbeitungseinrichtungen der Chiplabor-Kartusche aufweisen.

Ferner wird ein Verfahren zum Analysieren einer Probe biologischen Materials vorgestellt, wobei das Verfahren folgende Schritte aufweist:
Einbringen der Probe durch eine Einbringöffnung hindurch in eine Probenaufnahmekammer einer Chiplabor-Kartusche;
Leiten einer ersten Teilmenge der eingebrachten Probe durch einen ersten Probenauslass aus der Probenaufnahmekammer zu einer fluidisch mit dem ersten Probenauslass der Probenaufnahmekammer verbundenen, ersten Probenbearbeitungseinrichtung und zumindest einer zweiten Teilmenge der eingebrachten Probe durch zumindest einen zweiten Probenauslass aus der Probenaufnahmekammer zu einer fluidisch mit dem zweiten Probenauslass der Probenaufnahmekammer verbundenen und fluidisch von der ersten Probenbearbeitungseinrichtung getrennten, zweiten Probenbearbeitungseinrichtung;
Durchführen eines ersten Vorbereitungsprozesses und eines ersten Analyseprozesses an der ersten Teilmenge der Probe in der ersten Probenbearbeitungseinrichtung sowie eines zweiten Vorbereitungsprozesses und eines zweiten Analyseprozesses an der zweiten Teilmenge der Probe in der zweiten Probenbearbeitungseinrichtung; und
Auswerten zumindest eines ersten Analyten, der durch den ersten Vorbereitungsprozess und den ersten Analyseprozess in der ersten Probenbearbeitungseinrichtung aus der ersten Teilmenge der Probe gewonnen ist, und zumindest eines zweiten Analyten, der durch den zweiten Vorbereitungsprozess und den zweiten Analyseprozess in der zweiten Probenbearbeitungseinrichtung aus der zweiten Teilmenge der Probe gewonnen ist.

Das Verfahren kann in Verbindung mit einer Ausführungsform des vorstehend genannten mikrofluidischen Systems vorteilhaft ausgeführt werden, um eine Probe biologischen Materials zu analysieren. Der Schritt des Leitens und zusätzlich oder alternativ der Schritt des Durchführens können durch Steuern von Probenbearbeitungseinrichtungen der Chiplabor-Kartusche ausgeführt werden. Insbesondere können die Vorbereitungsprozesse und die Analyseprozesse parallel durchgeführt werden. Dabei können sich die Vorbereitungsprozesse und zusätzlich oder alternativ die Analyseprozesse zumindest teilweise hinsichtlich Prozessparametern unterscheiden.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen bzw. umzusetzen. Auch durch diese Ausführungsvariante der Erfindung in Form einer Vorrichtung kann die der Erfindung zugrunde liegende Aufgabe schnell und effizient gelöst werden.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Der hier vorgestellte Ansatz wird nachstehend anhand der beigefügten Zeichnungen beispielhaft näher erläutert. Es zeigen:
Fig. 1 ein Prozessschema zur Probenbearbeitung;
Fig. 2 ein Ablaufdiagramm eines Verfahrens gemäß einem Ausführungsbeispiel der vorliegenden Erfindung;
Fig. 3 eine schematische Darstellung eines mikrofluidischen Systems gemäß einem Ausführungsbeispiel der vorliegenden Erfindung;
Fig. 4 ein Prozessschema zur Probenbearbeitung gemäß einem Ausführungsbeispiel der vorliegenden Erfindung; und
Fig. 5 ein Prozessschema zur Probenbearbeitung gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

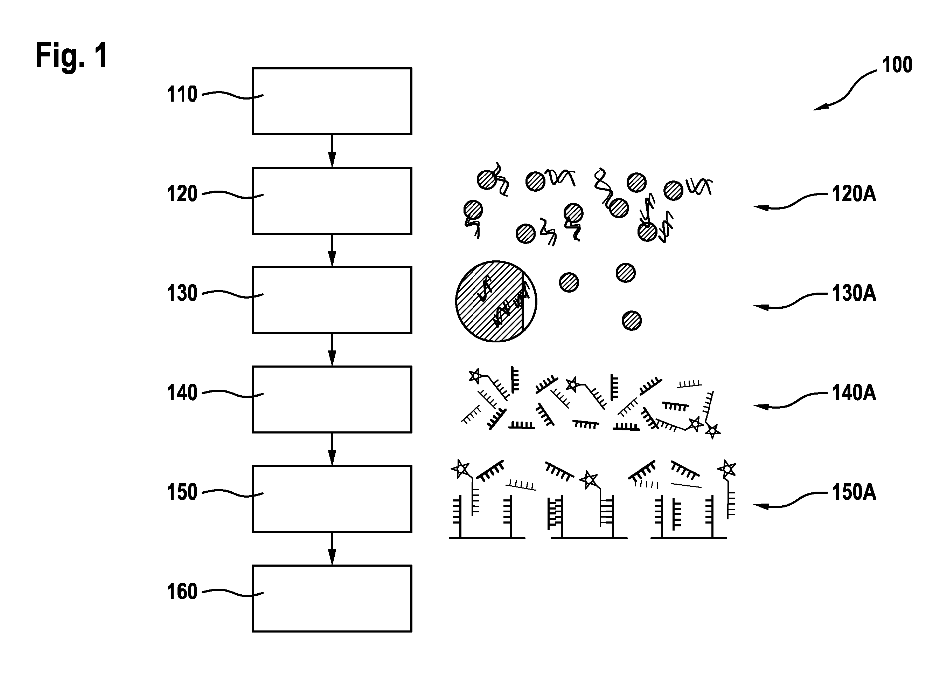

Fig. 1 zeigt ein Prozessschema 100 zur Probenbearbeitung. Hierbei stellt das Prozessschema 100 beispielsweise eine Polymerase-Kettenreaktion bzw. PCR (PCR, engl. Polymerase Chain Reaction) zur Bearbeitung einer Probe biologischen Materials im Rahmen eines Molekularassays dar.

In einem Block 110 erfolgt eine Probeneingabe. Anschließend wird in einem Block 120 eine Zelllyse bzw. ein Zellaufschluss durchgeführt, was in der Darstellung in Fig. 1 zusätzlich in einem dem Block 120 zugeordneten Schaubild 120A vereinfacht veranschaulicht ist. Danach erfolgt in einem Block 130 eine Aufreinigung von Nukleinsäuren bzw. eine DNA-Aufreinigung, was in der Darstellung in Fig. 1 wiederum zusätzlich in einem dem Block 130 zugeordneten Schaubild 130A vereinfacht veranschaulicht ist. Hiernach wird in einem Block 140 eine Amplifikation der Nukleinsäuren bzw. eine DNA-Amplifikation durchgeführt, was ebenfalls in der Darstellung in Fig. 1 zusätzlich in einem dem Block 140 zugeordneten Schaubild 140A vereinfacht veranschaulicht ist. Anschließend erfolgt in einem Block 150 eine Hybridisierung, was in der Darstellung in Fig. 1 zusätzlich in einem dem Block 150 zugeordneten Schaubild 150A vereinfacht veranschaulicht ist. Schließlich weist das Prozessschema 100 einen Block 160 des optischen Auslesens auf.

Hierbei repräsentiert der Block 120 der Zelllyse eine Stufe I, repräsentiert der Block 130 der DNA-Aufreinigung eine Stufe II, repräsentiert der Block 140 der DNA-Amplifikation eine Stufe III und repräsentiert der Block 150 der Hybridisierung eine Stufe IV der eigentlichen Polymerase-Kettenreaktion.

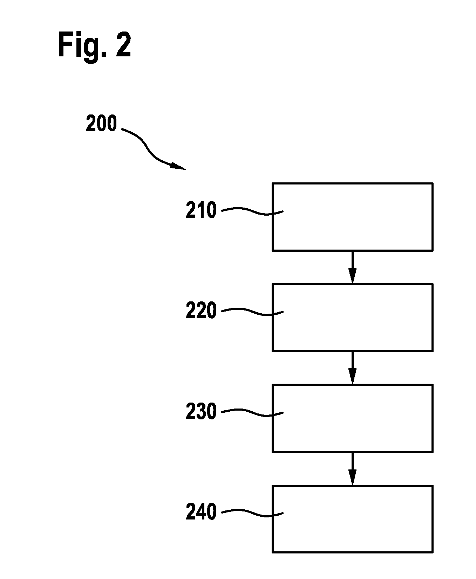

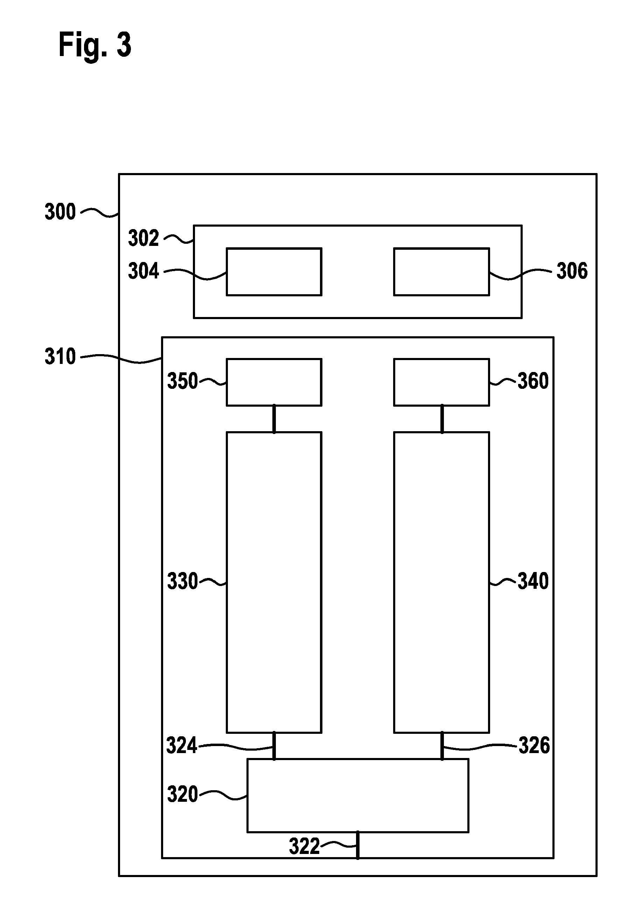

Fig. 2 zeigt ein Ablaufdiagramm eines Verfahrens 200 zum Analysieren einer Probe biologischen Materials gemäß einem Ausführungsbeispiel der vorliegenden Erfindung. Das Verfahren 200 ist unter Verwendung eines mikrofluidischen Systems, wie dem mikrofluidischen System aus Fig. 3 vorteilhaft ausführbar. Gezeigt sind von dem Verfahren 200 in Fig. 2 hierbei ein Schritt 210 des Einbringens, ein Schritt 220 des Leitens, ein Schritt 230 des Durchführens und ein Schritt 240 des Auswertens.

Im Schritt 210 des Einbringens wird die Probe durch eine Einbringöffnung hindurch in eine Probenaufnahmekammer einer Chiplabor-Kartusche eingebracht.

Nachfolgend wird im Schritt 220 des Leitens eine erste Teilmenge der eingebrachten Probe durch einen ersten Probenauslass aus der Probenaufnahmekammer zu einer fluidisch mit dem ersten Probenauslass der Probenaufnahmekammer verbundenen, ersten Probenbearbeitungseinrichtung geleitet. Auch wird im Schritt 220 des Leitens zumindest eine zweite Teilmenge der eingebrachten Probe durch zumindest einen zweiten Probenauslass aus der Probenaufnahmekammer zu einer fluidisch mit dem zweiten Probenauslass der Probenaufnahmekammer verbundenen, zweiten Probenbearbeitungseinrichtung geleitet, die fluidisch von der ersten Probenbearbeitungseinrichtung getrennt ist.

Danach werden im Schritt 230 des Durchführens ein erster Vorbereitungsprozess und ein erster Analyseprozess an der ersten Teilmenge der Probe in der ersten Probenbearbeitungseinrichtung durchgeführt. Ferner werden an der zweiten Teilmenge der Probe im Schritt 230 des Durchführens ein zweiter Vorbereitungsprozess sowie ein zweiter Analyseprozess in der zweiten Probenbearbeitungseinrichtung durchgeführt.

Schließlich wird im Schritt 240 des Auswertens zumindest ein erster Analyt ausgewertet, der durch den ersten Vorbereitungsprozess und den ersten Analyseprozess in der ersten Probenbearbeitungseinrichtung aus der ersten Teilmenge der Probe gewonnen ist. Im Schritt 240 des Auswertens wird auch zumindest ein zweiter Analyt ausgewertet, der durch den zweiten Vorbereitungsprozess und den zweiten Analyseprozess in der zweiten Probenbearbeitungseinrichtung aus der zweiten Teilmenge der Probe gewonnen ist.

Fig. 3 zeigt eine schematische Darstellung eines mikrofluidischen Systems 300 zum Analysieren einer Probe biologischen Materials gemäß einem Ausführungsbeispiel der vorliegenden Erfindung. Unter Verwendung des mikrofluidischen Systems 300 ist ein Verfahren wie das Verfahren aus Fig. 2 vorteilhaft ausführbar. Gezeigt sind von dem mikrofluidischen System 300 in Fig. 3 hierbei eine Auswerteeinrichtung 302, ein erster Sensor 304, ein zweiter Sensor 306, eine mikrofluidische Kartusche bzw. Chiplabor-Kartusche 310 zum Analysieren einer Probe biologischen Materials, eine Probenaufnahmekammer 320, eine Einbringöffnung 322, ein erster Probenauslass 324, ein zweiter Probenauslass 326, eine erste Probenbearbeitungseinrichtung 330, eine zweite Probenbearbeitungseinrichtung 340, eine erste Ergebniskammer 350 und eine zweite Ergebniskammer 360.

Das mikrofluidische System 300 weist die Auswerteeinrichtung 302 und die Chiplabor-Kartusche 310 auf. Die Auswerteeinrichtung 302 weist den ersten Sensor 304 und den zweiten Sensor 306 auf. Gemäß einem Ausführungsbeispiel kann die Auswerteeinrichtung 302 auch weniger oder mehr als die beiden Sensoren 300 3406 aufweisen. Die Chiplabor-Kartusche 310 weist die Probenaufnahmekammer 320, die Einbringöffnung 322, den ersten Probenauslass 324, den zweiten Probenauslass 326, die erste Probenbearbeitungseinrichtung 330, die zweite Probenbearbeitungseinrichtung 340, die erste Ergebniskammer 350 und die zweite Ergebniskammer 360 auf. Dabei ist die Chiplabor-Kartusche 310 in dem mikrofluidischen System 300 aufgenommen angeordnet.

Die Probenaufnahmekammer 320 der Chiplabor-Kartusche 310 weist die Einbringöffnung 322, den ersten Probenauslass 324 und den zweiten Probenauslass 326 auf. Dabei ist die Einbringöffnung 322 ausgeformt und ausgebildet, um ein Einbringen der Probe biologischen Materials in die Chiplabor-Kartusche 310 zu ermöglichen. Der erste Probenauslass 324 ist ausgeformt und ausgebildet, um ein Auslassen einer ersten Teilmenge der Probe aus der Probenaufnahmekammer 320 zu ermöglichen. Der zweite Probenauslass 326 ist ausgeformt und ausgebildet, um ein Auslassen einer zweiten Teilmenge der Probe aus der Probenaufnahmekammer 320 zu ermöglichen. Somit ist die Probenaufnahmekammer 320 ausgebildet, um eine eingebrachte Probe biologischen Materials aufzuteilen, um eine erste Teilmenge und eine zweite Teilmenge der Probe weiterzuverarbeiten.

Die erste Probenbearbeitungseinrichtung 330 ist an einem ersten Ende fluidisch mit dem ersten Probenauslass 324 der Probenaufnahmekammer 320 verbunden, insbesondere zum Aufnehmen der ersten Teilmenge der Probe. An einem zweiten Ende ist die erste Probenbearbeitungseinrichtung 330 fluidisch mit der ersten Ergebniskammer 350 verbunden, insbesondere zum Bereitstellen zumindest eines ersten Analyten. Somit erstreckt sich die erste Probenbearbeitungseinrichtung 330 zwischen dem ersten Probenauslass 324 und der ersten Ergebniskammer 350. Dabei ist die erste Probenbearbeitungseinrichtung 330 ausgebildet, um einen ersten Vorbereitungsprozess und einen ersten Analyseprozess an der ersten Teilmenge der Probe durchzuführen. Die erste Probenbearbeitungseinrichtung 330 ist somit auch ausgebildet, um aus der ersten Teilmenge der Probe zumindest einen ersten Analyten zu gewinnen.

Auch wenn es in Fig. 3 nicht explizit gezeigt ist, weist die erste Probenbearbeitungseinrichtung 330 hierzu Bearbeitungsmittel auf, die ausgebildet sind, um als ersten Vorbereitungsprozess zumindest einen Teilschritt des Aufbereitens, des Aufschließens und zusätzlich oder alternativ des Aufreinigens der ersten Teilmenge der Probe durchzuführen und zusätzlich oder alternativ als ersten Analyseprozess zumindest einen Teilschritt des Durchführens eines Immunassays, eines Molekularassays und zusätzlich oder alternativ einer Polymerasekettenreaktion an der ersten Teilmenge der Probe durchzuführen.

Die erste Ergebniskammer 350 ist ausgebildet, um den zumindest einen ersten Analyten, der durch den ersten Vorbereitungsprozess und den ersten Analyseprozess mittels der ersten Probenbearbeitungseinrichtung 330 aus der ersten Teilmenge der Probe gewinnbar bzw. gewonnen ist, zu sammeln bzw. zu beinhalten.

Die zweite Probenbearbeitungseinrichtung 340 ist an einem ersten Ende fluidisch mit dem zweiten Probenauslass 326 der Probenaufnahmekammer 320 verbunden, insbesondere zum Aufnehmen der zweiten Teilmenge der Probe. An einem zweiten Ende ist die zweite Probenbearbeitungseinrichtung 340 fluidisch mit der zweiten Ergebniskammer 360 verbunden, insbesondere zum Bereitstellen zumindest eines zweiten Analyten. Die zweite Probenbearbeitungseinrichtung 340 ist dabei fluidisch von der ersten Probenbearbeitungseinrichtung 330 getrennt angeordnet. Somit erstreckt sich die zweite Probenbearbeitungseinrichtung 340 zwischen dem zweiten Probenauslass 326 und der zweiten Ergebniskammer 360. Dabei ist die zweite Probenbearbeitungseinrichtung 340 ausgebildet, um einen zweiten Vorbereitungsprozess und einen zweiten Analyseprozess an der zweiten Teilmenge der Probe durchzuführen. Die zweite Probenbearbeitungseinrichtung 340 ist somit auch ausgebildet, um aus der zweiten Teilmenge der Probe zumindest einen zweiten Analyten zu gewinnen.

Auch wenn es in Fig. 3 nicht explizit gezeigt ist, weist die zweite Probenbearbeitungseinrichtung 340 hierzu Bearbeitungsmittel auf, die ausgebildet sind, um als zweiten Vorbereitungsprozess zumindest einen Teilschritt des Aufbereitens, des Aufschließens und zusätzlich oder alternativ des Aufreinigens der ersten Teilmenge der Probe durchzuführen und zusätzlich oder alternativ als zweiten Analyseprozess zumindest einen Teilschritt des Durchführens eines Immunassays, eines Molekularassays und zusätzlich oder alternativ einer Polymerasekettenreaktion an der ersten Teilmenge der Probe durchzuführen.

Die zweite Ergebniskammer 360 ist ausgebildet, um den zumindest einen zweiten Analyten, der durch den zweiten Vorbereitungsprozess und den zweiten Analyseprozess mittels der zweiten Probenbearbeitungseinrichtung 340 aus der zweiten Teilmenge der Probe gewinnbar bzw. gewonnen ist, zu sammeln bzw. zu beinhalten.

Somit sind die erste Probenbearbeitungseinrichtung 330 und die zweite Probenbearbeitungseinrichtung 340 hinsichtlich eines Fluidflusses relativ zueinander parallel geschaltet. Gemäß einem Ausführungsbeispiel kann sich der erste Vorbereitungsprozess von dem zweiten Vorbereitungsprozess in zumindest einem Teilschritt unterscheiden. Zusätzlich oder alternativ kann sich gemäß einem Ausführungsbeispiel der erste Analyseprozess von dem zweiten Analyseprozess in zumindest einem Teilschritt unterscheiden. Dabei kann die erste Probenbearbeitungseinrichtung 330 Bearbeitungsmittel aufweisen, die sich von den Bearbeitungsmitteln der zweiten Probenbearbeitungseinrichtung 340 unterscheiden können.

Der erste Vorbereitungsprozess und der erste Analyseprozess, die in der ersten Probenbearbeitungseinrichtung 330 durchgeführt werden, stellen beispielsweise einen ersten sogenannten Assay dar. Der zweite Vorbereitungsprozess und der zweite Analyseprozess, die in der zweiten Probenbearbeitungseinrichtung 340 durchgeführt werden, stellen beispielsweise einen zweiten Assay dar.

Bei einem Assay handelt es sich beispielsweise um einen molekulardiagnostische Assay bzw. Molekularassay, der ausgebildet ist, um ein Vorhandensein von Nukleinsäuren bzw. Erbinformation in Form von DNA oder RNA von Bakterien und Viren zu erkennen, oder um einen Protein-Assay bzw. Immunassay. Bei molekularen Assays werden prinzipiell Schritte einer Probenvorbereitung mit Zelllyse, einer DNA-Aufreinigung, einer DNA-Amplifikation, beispielsweise durch Polymerase-Kettenreaktion, und einer Hybridisierung durchlaufen. Eine Auftrennung eines Assayablaufs erfolgt gemäß Ausführungsbeispielen der vorliegenden Erfindung insbesondere vor der Aufreinigung, wobei die DNA-Sequenzen auf mehrere PCR-Stränge aufgeteilt und mittels unterschiedlicher PCR-Mixe vervielfältigt werden. Auch die Hybridisierung und ein Auslesen der Informationen erfolgt gemäß Ausführungsbeispielen der vorliegenden Erfindung parallel in mehreren, unterschiedlichen fluidischen Kammern der Probenbearbeitungseinrichtungen 330 und 340.

Die Auswerteeinrichtung 302 ist ausgebildet, um die mittels der Chiplabor-Kartusche 310 analysierte Probe, insbesondere optisch, auszuwerten. In einem in das mikrofluidische System 300 eingelegten Zustand der Chiplabor-Kartusche 310 ist der erste Sensor 304 der Auswerteeinrichtung 302 benachbart zu der ersten Ergebniskammer 350 angeordnet und ist der zweite Sensor 306 benachbart zu der zweiten Ergebniskammer 360 angeordnet. Dabei ist der erste Sensor 304 ausgebildet, um den zumindest einen ersten Analyten auszuwerten, der durch den ersten Vorbereitungsprozess und den ersten Analyseprozess mittels der ersten Probenbearbeitungseinrichtung 330 aus der ersten Teilmenge der Probe gewinnbar bzw. gewonnen und in der ersten Ergebniskammer 350 angeordnet ist. Dabei ist der zweite Sensor 306 ausgebildet, um den zumindest einen zweiten Analyten auszuwerten, der durch den zweiten Vorbereitungsprozess und den zweiten Analyseprozess mittels der zweiten Probenbearbeitungseinrichtung 340 aus der zweiten Teilmenge der Probe gewinnbar bzw. gewonnen und in der zweiten Ergebniskammer 360 angeordnet ist.

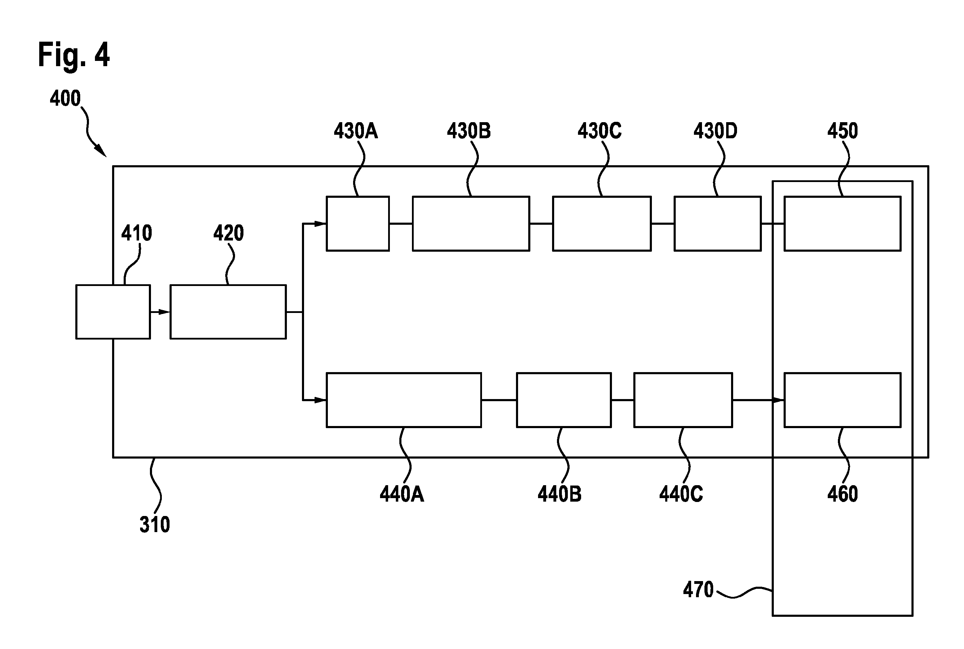

Fig. 4 zeigt ein Prozessschema zur Probenbearbeitung mittels einer Chiplabor-Kartusche 310 gemäß einem Ausführungsbeispiel der vorliegenden Erfindung. Bei der Chiplabor-Kartusche 310 handelt es sich beispielsweise um die in Fig. 3 dargestellte Chiplabor-Kartusche. Gezeigt sind die Chiplabor-Kartusche 310 und ein Prozessschema 400. Dabei weist das Prozessschema 400 zur Probenbearbeitung eine Mehrzahl von als Blöcke 410, 420, 430A, 430B, 430C, 430D, 440A, 440B, 440C, 450, 460 und 470 dargestellte Schritte bzw. Teilschritte auf. Die Schritte bzw. Teilschritte repräsentieren dabei Schritte bzw. Teilschritte eines Verfahrens wie dem Verfahren aus Fig. 2. Das Prozessschema 400 umfasst hierbei insbesondere den ersten Vorbereitungsprozess und den ersten Analyseprozess der ersten Probenbearbeitungseinrichtung sowie den zweiten Vorbereitungsprozess und den zweiten Analyseprozess der zweiten Probenbearbeitungseinrichtung der Chiplabor-Kartusche aus Fig. 3.

In dem Block 410 erfolgt eine Probeneingabe. Danach wird in dem Block 420 eine Probenaufbereitung, d. h. das Abwaschen und Lösen der Erreger in Lyseflüssigkeit, durchgeführt. Nach dem Block 420 verzweigt sich das Prozessschema 400 in einen ersten Pfad mit den Blöcken 430A, 430B, 430C, 430D und 450, die eine Probenbearbeitung mittels der ersten Probenbearbeitungseinrichtung der Chiplabor-Kartusche aus Fig. 3 repräsentieren, und in einem zweiten Pfad mit den Blöcken 440A, 440B, 440C und 460, die eine Probenbearbeitung mittels der zweiten Probenbearbeitungseinrichtung der Chiplabor-Kartusche aus Fig. 3 repräsentieren.

In dem Block 430A wird eine Lyse bzw. ein Aufschluss der Probe vorgenommen. Anschließend erfolgt in dem Block 430B eine DNA-Aufreinigung der lysierten bzw. aufgeschlossenen Probe. In dem Block 430C wird eine DNA-Amplifikation durchgeführt, gefolgt von einer Hybridisierung in dem Block 430D. Anschließend erfolgt in dem Block 450 eine optische Auswertung. Somit wird in den Blöcken 430A, 430B, 430C und 430D ein Molekularassay durchgeführt.

In dem Block 440A erfolgt ein zweiter Schritt eines Immunassays. Anschließend werden in dem Block 440B ein dritter Schritt des Immunassays sowie in dem darauf folgenden Block 440C ein vierter Schritt des Immunassays durchgeführt. Schließlich erfolgt in dem Block 460 eine optische Auswertung. Beispielsweise können die Schritte des Immunassays die folgenden Arbeitsschritte aufweisen:
- Probe wird über Trägeroberfläche mit Antikörpern in Kammer gespült.
- Auswaschen
- Konjugat in Kammer spülen
- Waschen
- Auswerten der Reaktion

Die Schritte bzw. Teilschritte der Blöcke 410 bis 460 sind auf der Chiplabor-Kartusche 310 ausführbar. Der Block 470 umfasst die Blöcke 450 und 460 der optischen Auswertung und betrifft eine Aggregation von Analysewerten in einer externen Diagnoseeinheit.

Anders ausgedrückt zeigt Fig. 4 eine schematische Darstellung einer Probenverarbeitung. Nach Eingabe des Probenmaterials in dem Block 410 wird die Probe in zwei fluidische Pfade aliquotiert. Dabei kann durch verschiedene Nachweismethoden ein Analyt bzw. Zielmolekül aus zwei unterschiedlichen Stoffklassen stammen, wie beispielsweise DNA und Protein. Es erfolgt gemäß dem Prozessschema 400 eine Prozessierung bzw. Durchführung von zwei Arten von Assays parallel auf einer Chiplabor-Kartusche 310. Vorteilhaft ist eine solche parallele Prozesssierung von unterschiedlichen Assays mit einer Probe, weil durch eine parallele Analyse eine medizinische Aussagekraft gesteigert werden kann. So kann mittels einer Chiplabor-Kartusche 310, die gemäß dem Prozessschema 400 beispielsweise zum Nachweis von sepsisverursachenden Pathogenen ausgelegt ist, mit einer Blutprobe in einem Pfad eine Proteinanalyse auf Procalcitonin (PCT, ein Prohormon und relevanter Marker in der Diagnostik von Sepsis) und in einem anderen Pfad eine DNA-Analyse der Pathogene durchgeführt werden. Ein Arzt erhält somit Informationen, ob ein Patient bereits das Prohormon gebildet hat, und auch detaillierte Informationen zu einem krankheitsverursachenden Erreger.

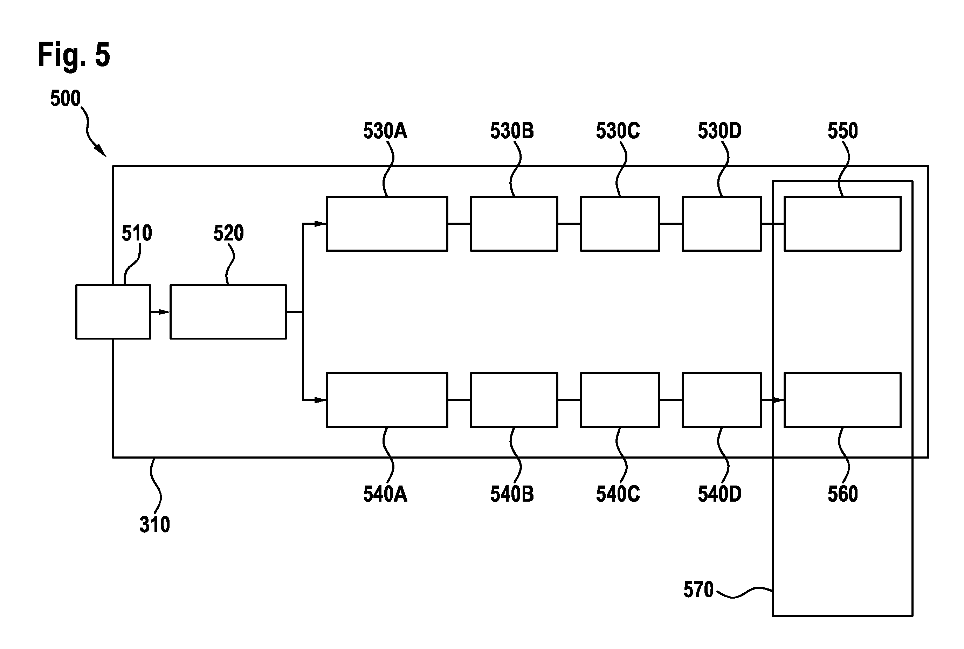

Fig. 5 zeigt ein Prozessschema zur Probenbearbeitung mittels einer Chiplabor-Kartusche 310 gemäß einem Ausführungsbeispiel der vorliegenden Erfindung. Bei der Chiplabor-Kartusche 310 handelt es sich beispielsweise um die in Fig. 3 dargestellte Chiplabor-Kartusche. Gezeigt sind die Chiplabor-Kartusche 310 und ein Prozessschema 500. Dabei weist das Prozessschema 500 zur Probenbearbeitung eine Mehrzahl von eine Mehrzahl von als Blöcke 510, 520, 530A, 530B, 530C, 530D, 540A, 540B, 540C, 540D, 550, 560 und 570 dargestellte Schritte bzw. Teilschritte auf. Die Schritte bzw. Teilschritte repräsentieren dabei Schritte bzw. Teilschritte eines Verfahrens wie dem Verfahren aus Fig. 2. Das Prozessschema 500 umfasst hierbei insbesondere den ersten Vorbereitungsprozess und den ersten Analyseprozess der ersten Probenbearbeitungseinrichtung sowie den zweiten Vorbereitungsprozess und den zweiten Analyseprozess der zweiten Probenbearbeitungseinrichtung der Chiplabor-Kartusche aus Fig. 3.

In dem Block 510 erfolgt eine Probeneingabe. Danach wird in dem Block 520 eine Probenaufbereitung durchgeführt. Nach dem Block 520 verzweigt sich das Prozessschema 500 in einen ersten Pfad mit den Blöcken 530A, 530B, 530C, 530D und 550, die eine Probenbearbeitung mittels der ersten Probenbearbeitungseinrichtung der Chiplabor-Kartusche aus Fig. 3 repräsentieren, und in einem zweiten Pfad mit den Blöcken 540A, 540B, 540C, 540D und 560, die eine Probenbearbeitung mittels der zweiten Probenbearbeitungseinrichtung der Chiplabor-Kartusche aus Fig. 3 repräsentieren.

In dem Block 530A wird eine erste Art einer Lyse bzw. eines Aufschlusses der Probe vorgenommen, beispielsweise enzymatisch. Anschließend erfolgt in dem Block 530B eine DNA-Aufreinigung der lysierten bzw. aufgeschlossenen Probe. In dem Block 530C wird eine DNA-Amplifikation durchgeführt, gefolgt von einer Hybridisierung in dem Block 530D. Anschließend erfolgt in dem Block 550 eine optische Auswertung. Somit wird in den Blöcken 530A, 530B, 530C und 530D ein Molekularassay durchgeführt.

In dem Block 540A wird eine zweite Art einer Lyse bzw. eines Aufschlusses der Probe vorgenommen, beispielsweise mechanisch. Anschließend erfolgt in dem Block 540B eine DNA-Aufreinigung der lysierten bzw. aufgeschlossenen Probe. In dem Block 540C wird eine DNA-Amplifikation durchgeführt, gefolgt von einer Hybridisierung in dem Block 540D. Anschließend erfolgt in dem Block 560 eine optische Auswertung. Somit wird in den Blöcken 540A, 540B, 540C und 540D ein Molekularassay durchgeführt.

Die Schritte bzw. Teilschritte der Blöcke 510 bis 560 sind auf der Chiplabor-Kartusche 310 ausführbar. Der Block 570 umfasst die Blöcke 550 und 560 der optischen Auswertung und betrifft einen Vergleich von Messwerten in einer externen Diagnoseeinheit.

Anders ausgedrückt zeigt Fig. 5 einen schematischen Aufbau einer Chiplabor-Kartusche 310 mit unterschiedlichen Lyseverfahren in den Blöcken 530A und 540A beispielsweise zur Unterscheidung von toten und lebenden Erregern in einer Patientenprobe. Es erfolgt gemäß dem Prozessschema 500 eine Prozessierung einer Probe mit zwei unterschiedlichen Lyseverfahren auf einer Chiplabor-Kartusche 310. Hierbei wird eine Probe vor den Blöcken 530A und 540A der Lyse aufgeteilt. Die Probe durchläuft damit zwei unterschiedliche Lyseverfahren den Blöcken 530A und 540A. So werden beispielsweise in einem Lyseverfahren tote Erregerzellen vernichtet und nur die Zellmembran aktiver Zellen aufgebrochen. Im anderen Lyseverfahren werden Zellwände aktiver und abgestorbener Erreger lysiert und DNA freigesetzt. Erfolgt nun eine PCR und Analyse der DNA-Sequenzen verteilt in den beiden Pfaden bzw. Fluidsträngen, so werden in einem Pfad nur die lebenden Erreger erkannt, während in dem anderen Pfad die lebenden und die toten Erreger erkannt werden. In der anschließenden Auswertung in den Blöcken 550, 560 und 570 kann daher unterschieden werden, welche Erreger bereits abgestorben sind und welche noch aktiv sind. Eine solche Information kann für einen Arzt von Vorteil sein, um einen Therapieverlauf zu verfolgen.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Chiplabor-Kartusche (310) für ein mikrofluidisches System (300) zum Analysieren einer Probe biologischen Materials, wobei die Chiplabor-Kartusche (310) folgende Merkmale aufweist:
eine Probenaufnahmekammer (320), die eine Einbringöffnung (322) zum Einbringen einer Probe biologischen Materials in die Chiplabor-Kartusche (310), einen ersten Probenauslass (324) zum Auslassen einer ersten Teilmenge der Probe aus der Probenaufnahmekammer (320) und zumindest einen zweiten Probenauslass (326) zum Auslassen zumindest einer zweiten Teilmenge der Probe aus der Probenaufnahmekammer (320) aufweist;
eine erste Probenbearbeitungseinrichtung (330), die fluidisch mit dem ersten Probenauslass (324) der Probenaufnahmekammer (320) verbunden ist, wobei die erste Probenbearbeitungseinrichtung (330) ausgebildet ist, um einen ersten Vorbereitungsprozess und einen ersten Analyseprozess an der ersten Teilmenge der Probe durchzuführen; und
eine zweite Probenbearbeitungseinrichtung (340), die fluidisch mit dem zweiten Probenauslass (326) der Probenaufnahmekammer (320) verbunden und fluidisch von der ersten Probenbearbeitungseinrichtung (330) getrennt ist, wobei die zweite Probenbearbeitungseinrichtung (340) ausgebildet ist, um einen zweiten Vorbereitungsprozess und einen zweiten Analyseprozess an der zweiten Teilmenge der Probe durchzuführen.

2. Chiplabor-Kartusche (310) gemäß Anspruch 1, **dadurch gekennzeichnet, dass** die erste Probenbearbeitungseinrichtung (330) Bearbeitungsmittel aufweist, die ausgebildet sind, um als ersten Vorbereitungsprozess zumindest einen Teilschritt des Aufbereitens, Aufschließens und/oder Aufreinigens der ersten Teilmenge der Probe durchzuführen und/oder als ersten Analyseprozess zumindest einen Teilschritt des Durchführens eines Immunassays, eines Molekularassays und/oder einer Polymerasekettenreaktion an der ersten Teilmenge der Probe durchzuführen.

3. Chiplabor-Kartusche (310) gemäß einem der vorangegangenen Ansprüche, **dadurch gekennzeichnet, dass** die zweite Probenbearbeitungseinrichtung (340) Bearbeitungsmittel aufweist, die ausgebildet sind, um als zweiten Vorbereitungsprozess zumindest einen Teilschritt des Aufbereitens, Aufschließens und/oder Aufreinigens der zweiten Teilmenge der Probe durchzuführen und/oder als zweiten Analyseprozess zumindest einen Teilschritt des Durchführens eines Immunassays, eines Molekularassays und/oder einer Polymerasekettenreaktion an der zweiten Teilmenge der Probe durchzuführen.

4. Chiplabor-Kartusche (310) gemäß einem der vorangegangenen Ansprüche, **dadurch gekennzeichnet, dass** sich der erste Vorbereitungsprozess von dem zweiten Vorbereitungsprozess in zumindest einem Teilschritt unterscheidet und/oder sich der erste Analyseprozess von dem zweiten Analyseprozess in zumindest einem Teilschritt unterscheidet.

5. Chiplabor-Kartusche (310) gemäß einem der vorangegangenen Ansprüche, **gekennzeichnet durch** eine erste Ergebniskammer (350) zum Beinhalten zumindest eines ersten Analyten, der **durch** den ersten Vorbereitungsprozess und den ersten Analyseprozess mittels der ersten Probenbearbeitungseinrichtung (330) aus der ersten Teilmenge der Probe gewinnbar ist, und/oder eine zweite Ergebniskammer (360) zum Beinhalten zumindest eines zweiten Analyten, der **durch** den zweiten Vorbereitungsprozess und den zweiten Analyseprozess mittels der zweiten Probenbearbeitungseinrichtung (340) aus der zweiten Teilmenge der Probe gewinnbar ist.

6. Mikrofluidisches System (300) zum Analysieren einer Probe biologischen Materials, wobei das mikrofluidische System (300) folgende Merkmale aufweist:
eine Chiplabor-Kartusche (310) gemäß einem der vorangegangenen Ansprüche; und
eine Auswerteeinrichtung (302) zum Auswerten der mittels der Chiplabor-Kartusche (310) analysierten Probe, wobei die Auswerteeinrichtung (302) ausgebildet ist, um zumindest einen ersten Analyten, der durch den ersten Vorbereitungsprozess und den ersten Analyseprozess mittels der ersten Probenbearbeitungseinrichtung (330) aus der ersten Teilmenge der Probe gewinnbar ist, und zumindest einem zweiten Analyten, der durch den zweiten Vorbereitungsprozess und den zweiten Analyseprozess mittels der zweiten Probenbearbeitungseinrichtung (340) aus der zweiten Teilmenge der Probe gewinnbar ist, auszuwerten.

7. Verfahren (200) zum Analysieren einer Probe biologischen Materials, wobei das Verfahren (200) folgende Schritte aufweist:
Einbringen (210) der Probe durch eine Einbringöffnung (322) hindurch in eine Probenaufnahmekammer (320) einer Chiplabor-Kartusche (310);
Leiten (220) einer ersten Teilmenge der eingebrachten Probe durch einen ersten Probenauslass (324) aus der Probenaufnahmekammer (320) zu einer fluidisch mit dem ersten Probenauslass (324) der Probenaufnahmekammer (320) verbundenen, ersten Probenbearbeitungseinrichtung (330) und zumindest einer zweiten Teilmenge der eingebrachten Probe durch zumindest einen zweiten Probenauslass (326) aus der Probenaufnahmekammer (320) zu einer fluidisch mit dem zweiten Probenauslass (326) der Probenaufnahmekammer (320) verbundenen und fluidisch von der ersten Probenbearbeitungseinrichtung (330) getrennten, zweiten Probenbearbeitungseinrichtung (340);
Durchführen (230) eines ersten Vorbereitungsprozesses und eines ersten Analyseprozesses an der ersten Teilmenge der Probe in der ersten Probenbearbeitungseinrichtung (330) sowie eines zweiten Vorbereitungsprozesses und eines zweiten Analyseprozesses an der zweiten Teilmenge der Probe in der zweiten Probenbearbeitungseinrichtung (340); und
Auswerten (240) zumindest eines ersten Analyten, der durch den ersten Vorbereitungsprozess und den ersten Analyseprozess in der ersten Probenbearbeitungseinrichtung (330) aus der ersten Teilmenge der Probe gewonnen ist, und zumindest eines zweiten Analyten, der durch den zweiten Vorbereitungsprozess und den zweiten Analyseprozess in der zweiten Probenbearbeitungseinrichtung (340) aus der zweiten Teilmenge der Probe gewonnen ist.

8. Vorrichtung, die ausgebildet ist, um alle Schritte eines Verfahrens (200) gemäß Anspruch 7 durchzuführen und/oder anzusteuern.

9. Computerprogramm, das dazu eingerichtet ist, alle Schritte eines Verfahrens (200) gemäß Anspruch 7 durchzuführen und/oder anzusteuern.

10. Maschinenlesbares Speichermedium mit einem darauf gespeicherten Computerprogramm nach Anspruch 9.
